**Europäisches Patentamt**

**European Patent Office**

**Office européen des brevets**

(11) Publication number : **0 504 980 B1**

(12) **EUROPEAN PATENT SPECIFICATION**

(45) Date of publication of patent specification :
01.06.94 Bulletin 94/22

(21) Application number : **92200690.3**

(22) Date of filing : **11.03.92**

(51) Int. Cl.⁵ : **B01J 21/12, C07C 41/06, C07C 41/44**

(54) Process for the selective catalytic decomposition of alkyl ethers and alcohols in a feedstock originating from the synthesis of methyl tert-amyl ether.

(30) Priority : **22.03.91 IT MI910779**

(43) Date of publication of application :
23.09.92 Bulletin 92/39

(45) Publication of the grant of the patent :
01.06.94 Bulletin 94/22

(84) Designated Contracting States :
AT BE DE DK ES FR GB GR NL SE

(56) References cited :
EP-A- 0 015 513
EP-A- 0 068 514
EP-A- 0 082 447
EP-A- 0 340 868

(73) Proprietor : **SNAMPROGETTI S.p.A.**
Corso Venezia 16
I-20121 Milan (IT)

(72) Inventor : **Ancillotti, Francesco**
Via Agadir 14/C
I-20097 San Donato Milanese (Milan) (IT)

(74) Representative : **De Carli, Erberto et al**
ING. BARZANO & ZANARDO MILANO S.p.A.
Via Borgonuovo, 10
I-20121 Milano (IT)

EP 0 504 980 B1

## Description

This invention relates to a process for the selective catalytic decomposition of alkyl ethers and alcohols in a feedstock originating from the synthesis of methyl tert- amyl ether (TAME). The invention relates in particular to a process for purifying a $C_5$ olefin cut originating from TAME synthesis to make it suitable for subsequent alkylation with isobutane. The process consists of catalytic treatment for removing oxygenated compounds which poison the alkylation catalyst.

It is well known that when methanol is present, branched unsaturated olefinic $C_5$ hydrocarbons (isoamylenes) give rise to the formation of TAME, the reaction being catalyzed by Lewis acids, mineral acids and organic acids (as described for example in U.K. Patent No. 1 506 596). A suitable refinery stream for TAME production is a $C_5$ fraction from catalytic cracking containing only small quantities of $C_4$ and $C_5$+ hydrocarbons.

TAME is used as an octane booster and can either be left in the reaction products or separated by distillation as a bottom product. In this latter case the remaining hydrocarbon products can be effectively used in the alkylation reaction provided they are of high purity (in particular, the oxygenated products must be contained at very low level).

In addition to TAME, the oxygenated products which may remain in said fraction are the tertiary amyl alcohol which forms as a by-product in TAME synthesis, and in particular MTBE which forms because of the isobutene present in small quantities in the $C_5$ cut and which because of its boiling point is difficult to separate from a $C_5$ fraction. The total removal of $C_4$ hydrocarbons from the $C_5$ cut could be a solution, but this would be very costly.

A catalyst system has now been found which allows selective decomposition of the oxygenated components present in products originating from TAME synthesis, so making the $C_5$ feedstock suitable for feeding to alkylation.

The process of the invention has high selectivity both with regard to the content of oxygenated components and with regard to the formation of heavy products, which would be detrimental to selectivity and would have no suitable use.

The present invention presents two simultaneous innovative aspects:

- The use of chemical methods (destructive catalytic elimination) to separate the undesirable oxygenated components instead of the use of physical methods such as extraction or distillation. Indeed, given the low content of oxygenated components to be eliminated, it is by no means certain that the result could be attained using the aforesaid conventional physical methods.
- The use of a highly selective catalyst, which eliminates the oxygenated components by reducing then to a level compatible with the alkylation reaction, while maintaining at a very low level the parallel oligomerization reaction or formation of $C_6$+ compounds, which as such would only reduce the reaction selectivity, being of low value.

The process for the decomposition of alkyl ethers and alcohols in a feedstock originating from TAME synthesis, according to the present invention, consists of reacting said feedstock in the presence of a catalyst consisting of silica modified by the addition of alumina in a quantity of between 0.1 and 1.5% by weight of the silica, operating at a temperature of between 200 and 250°C and at a space velocity (LHSV) of between 4 and 15 $h^{-1}$. The decomposition is conducted preferably at an operating pressure of between 1 and 2 bars.

The catalyst can be easily prepared, starting from a preformed silica of the required purity (this being a commercially available product), thus limiting the preparation to impregnation, drying and calcining.

The impregnation is effected with a solution of an aluminium salt (such as the nitrate or isopropylate) to provide the final desired alumina content.

It is however advisable to use a high purity silica, ie a silica with an $Na_2O$ content not exceeding 0.12% by weight, an $SO_4$ content not exceeding 0.15% by weight and an $Al_2O_3$ content not exceeding 0.30% by weight.

The catalysts used in said process are neither toxic nor corrosive, and can operate in the presence of water without giving rise to the formation of acid products.

In addition they are stable with time for many thousands of hours and can be easily regenerated.

The method of operation and the advantages of the process according to the present invention will be more apparent from the following examples, which are provided to illustrate the invention but without being limitative thereof.

EXAMPLES

The research was conducted on a synthetic mixture reproducing the composition of the effluent from TAME synthesis. Its composition is as follows:

| Component | % by weight |
|---|---|
| $C_4$ | 0.016 |
| Isopentane | 45.933 |
| Normal pentane | 5.564 |
| 2-methyl-2-butene | 6.665 |
| 2-methyl-1-butene | 1.268 |
| 1-pentene | 35.640 |
| MTBE | 3.634 |
| Tert-amyl alcohol (TAA) | 0.583 |
| TAME | 0.547 |
| $C_6-C_{10}$ | 0.150 |

The catalytic tests were conducted in a flow microreactor of plug-flow type containing 1 cm³ of catalyst of suitable particle size, 30-40 mesh (0.4-0.5 mm).

EXAMPLE 1

The alumina-modified silica catalyst was prepared in the following manner:
10 g of high purity silica of the following composition:

| | | | |
|---|---|---|---|
| $Na_2O$ | | 0.05% by weight | |
| $SO_4$ | | 0.15 | " |
| $Al_2O_3$ | | 0.30 | " |
| $SiO_2$ | remainder to | 100 | " |

are treated with an aqueous solution containing 0.368 g of aluminium nitrate (added $Al_2O_3$ = 0.5% by weight of the silica).

The material obtained is dried slowly at 120°C for 3 hours, then calcined at 500°C for 4 hours.

1 cm³ of catalyst prepared in this manner (consisting of 0.5% $Al_2O_3$ on high-purity silica) is placed in the microreactor and heated to a temperature of 240°C. The pressure is 1.6 bars. The reaction mixture is then fed at LHSV = 4, the results obtained being shown in Table 1.

EXAMPLE 2

Without any intermediate regeneration, the catalyst of Example 1 is adjusted to a temperature of 230°C and the LHSV raised to 5.6. The pressure is maintained at 1.6 bars. Product analysis is conducted after stabilization. The results are shown in Table 1.

EXAMPLE 3

With the other operating conditions of the preceding examples unaltered, the LHSV is raised to 10. Product analysis is conducted after stabilization. The results are shown in Table 1.

EXAMPLE 4

With the other operating conditions of the preceding example unaltered, the LHSV is raised to 11. The test results are shown in Table 1.

EXAMPLE 5

The test is continued by raising the temperature to 235°C and setting the LHSV at 10. Analysis of the reaction products is conducted after stabilization. The results are shown in Table 1.

EXAMPLE 6

The test is continued by further raising the temperature to 240°C and the LHSV to 11.6. Product analysis is conducted after stabilization. The results are shown in Table 1.

EXAMPLE 7 - Comparative

The catalyst of Example 1 is placed in the microreactor and raised to a temperature of 240°C. The operating pressure is 1.6 bars. The reaction mixture is fed at an LHSV of 0.5. The results obtained are shown in Table 1.

EXAMPLE 8 - Comparative

The catalyst of Example 1 is placed in the microreactor and raised to a temperature of 240°C. The operating pressure is 1.6 bars. The reaction mixture is fed at an LHSV of 24.0. The results obtained are shown in Table 1.

EXAMPLE 9 - Comparative

The catalyst of Example 1 is placed in the microreactor and raised to a temperature of 350°C. The operating pressure is 1.6 bars. The reaction mixture is fed at an LHSV of 11.5. The results obtained are shown in Table 1.

From the data of Table 1 it can he seen that very high space velocities (LHSV greater than 15) (Example 8) do not result in complete methyl-tertiary-butyl-ether (MTBE) decomposition, making the product unsuitable for alkylation.

Too low a space velocity (LHSV less than 4) (Example 7) or too high a temperature (exceeding 250°C) (Example 9) result in a considerable reduction in olefin selectivity due to the greater formation of by-products from side reactions.

## TABLE 1

| EXAMPLE | TEMP °C | LHSV | MTBE ppm | TAA ppm | TAME ppm | C4 SELECTIVITY | C5 SELECTIVITY | % C6+ | DME ppm |
|---|---|---|---|---|---|---|---|---|---|
| 1 | 240 | 4 | 16 | - | - | 92.5 | 99.1 | 1.26 | 1360 |
| 2 | 230 | 5.6 | 15 | - | - | 94.5 | 99.5 | 0.73 | 710 |
| 3 | 230 | 10 | 12 | - | - | 99.1 | 99.7 | 0.48 | 390 |
| 4 | 230 | 11 | 13 | - | - | 99.1 | 99.9 | 0.27 | 160 |
| 5 | 235 | 10 | 9 | - | - | 99.9 | 99.9 | 0.29 | 210 |
| 6 | 240 | 11.6 | 7 | - | - | 99.8 | 99.8 | 0.34 | 350 |
| 7 | 240 | 0.5 | - | - | - | 70.9 | 97.7* | 2.30 | |
| 8 | 240 | 24.0 | 100 | 10 | 10 | 99.1 | 99.9 | 0.30 | |
| 9 | 350 | 11.5 | - | - | - | 83.7 | 95.0* | 4.60 | |

$C_4$ selectivity means the ratio of $C_4$s which have remained unaltered to $C_4$s present in the feed.

$C_5$ selectivity means the ratio of $C_5$s which have remained unaltered to $C_5$s present in the feed.

%$C_6$+ means the percentage of heavy products formed during the reaction.

(*) in these cases the $C_5$ selectivity also includes the isopentane and normal pentane which in these tests, in contrast to the others, are formed in some quantity (2-3%, so that the true olefin selectivity is 86.0% for Example **7** and 87.0% for Example 9).

## Claims

1. A process for the selective catalytic decomposition of alkyl ethers and alcohols in a feedstock originating from the synthesis of methyl tert-amyl ether (TAME), characterised by reacting said feedstock in the presence of a catalyst consisting of silica modified by the addition of alumina in a quantity of between 0.1 and 1.5% by weight of the silica, operating at a temperature of between 200 and 250°C and at a space velocity (LHSV) of between 4 and 15 h$^{-1}$.

2. A process as claimed in claim 1, wherein the feedstock is reacted at a pressure of between 1 and 2 bars.

3. A process as claimed in claim 1, wherein the silica forming the catalyst is of high purity.

4. A process as claimed in claim 3, wherein the high purity silica has an Na$_2$O content not exceeding 0.12% by weight, an SO$_4$ content not exceeding 0.15% by weight and an Al$_2$O$_3$ content not exceeding 0.30% by weight.

**Patentansprüche**

1. Verfahren zur selektiven katalytischen Zersetzung von Alkylethern und Alkoholen in einem aus der Synthese von Methyl-tert.amylether (TAME) stammenden Einsatzmaterial, dadurch gekennzeichnet, daß dieses Einsatzmaterial in Gegenwart eines aus Siliziumdioxid bestehenden Katalysators, der durch Zugabe von Aluminiumoxid in einer Menge zwischen 0,1 und 1,5 Gew.-% des Siliziumdioxids modifiziert worden ist, zur Umsetzung gebracht wird, wobei bei einer Temperatur zwischen 200 und 250°C und bei einer Raumgeschwindigkeit (LHSV) zwischen 4 und 15 . h$^{-1}$ gearbeitet wird.

2. Verfahren nach Anspruch 1, worin das Einsatzmaterial bei einem Druck zwischen 1 und 2 bar zur Reaktion gebracht wird.

3. Verfahren nach Anspruch 1, worin das den Katalysator bildende Siliziumdioxid von hoher Reinheit ist.

4. Verfahren nach Anspruch 3, worin das hochreine Siliziumdioxid einen 0,12 Gew.-% nicht überschreitenden Na$_2$O-Gehalt, einen 0,15 Gew.-% nicht überschreitenden SO$_4$-Gehalt und einen 0,30 Gew.-% nicht überschreitenden Al$_2$O$_3$-Gehalt aufweist.

**Revendications**

1. Procédé de décomposition catalytique sélective d'oxydes d'alkyle et d'alcools, présents dans une charge provenant de la synthèse de l'oxyde de méthyle et de tert.-amyle (TAME), caractérisé en ce que l'on fait réagir ladite charge en présence d'un catalyseur constitué de silice modifiée par addition d'alumine en une quantité comprise entre 0,1 et 1,5% en poids par rapport à la silice, en opérant à une température comprise entre 200 et 250°C et à une vitesse spatiale (LHSV) comprise entre 4 et 15 h$^{-1}$.

2. Procédé selon la revendication 1, dans lequel on fait réagir la charge à une pression comprise entre 1 et 2 bars.

3. Procédé selon la revendication 1, dans lequel la silice formant le catalyseur a une pureté élevée.

4. Procédé selon la revendication 3, dans lequel la silice de pureté élevée présente une teneur en Na$_2$O ne dépassant pas 0,12% en poids, une teneur en SO$_4$ ne dépassant pas 0,15% en poids, et une teneur en Al$_2$O$_3$ ne dépassant pas 0,30% en poids.